# EUROPEAN PATENT SPECIFICATION

(11) **EP 4 130 532 B1**
(45) Date of publication and mention of the grant of the patent: **24.04.2024**
(21) Application number: 21189584.2
(22) Date of filing: 04.08.2021
(51) Int. Cl.: C07C 51/44, G01F 11/24

(54) **DEVICE FOR THE PRODUCTION OF BIOPOLYMERS FROM BIOMASS**
VORRICHTUNG ZUR HERSTELLUNG VON BIOPOLYMEREN AUS BIOMASSE
DISPOSITIF POUR LA PRODUCTION DE BIOPOLYMÈRES À PARTIR DE BIOMASSE

(43) Date of publication of application: 08.02.2023
(73) Proprietor: Technische Hochschule Rosenheim, 83024 Rosenheim (DE)
(72) Inventor: STRÜBBE, Nicole, 83024 Rosenheim (DE); MUSCAT, Dirk, 83024 Rosenheim (DE); ZENZ, Vitus, 83024 Rosenheim (DE)
(74) Representative: Maiwald GmbH

(56) References cited:
- DE-C- 349 646
- US-A- 4 128 051
- US-A- 5 859 263
- US-A1- 2005 067 729
- US-A1- 2009 158 664
- US-A1- 2017 121 116
- VERRECK G ET AL: "The effect of pressurized carbon dioxide as a plasticizer and foaming agent on the hot melt extrusion process and extrudate properties of pharmaceutical polymers", THE JOURNAL OF SUPERCRITICAL FLUIDS, ELSEVIER, AMSTERDAM, NL, vol. 38, no. 3, 1 October 2006 (2006-10-01), pages 383-391, XP024910238, ISSN: 0896-8446, DOI: 10.1016/J.SUPFLU.2005.11.022 [retrieved on 2006-10-01]
- DEUTZENBERG G ET AL: "Bio based fuels and fuel additives from lignocellulose feedstock via the production of levulinic acid and furfural", HOLZFORSCHUNG: INTERNATIONAL JOURNAL OF THE BIOLOGY, CHEMISTRY, PHYSICS AND TECHNOLOGY OF WOOD, WALTER DE GRUYTER GMBH & CO., BERLIN, DE, vol. 65, 1 January 2011 (2011-01-01), pages 439-451, XP008174089, ISSN: 0018-3830, DOI: 10.1515/HF.2011.081

## Description

### Technical field

The present invention relates to an apparatus for a high pressure extruding processing, a pressure lock device to be used with the apparatus and a method for production of biopolymers from biomass, in particular for the production of levulinic acid from woody residues.

### Technical Background

For processes taking place under high pressure conditions, devices for introducing material in pressure processes, for example in pressure chambers of plants for gasification of biomass and the like, are known. Such devices are used whenever material is to be brought from an area with lower pressure level into a closed area with higher pressure level. The challenge is carrying out introducing material without significantly affecting the higher pressure level and without causing a backflow of gases.

Vessel locks may be used to introduce material, in particular solids, from a vessel into pressurized volumes. A vessel lock may be coupled to a vessel that can be filled with material and optionally pressurized. The vessel is filled with the material while the vessel is separated and sealed from the process pressure in the pressurized volume. A container of the vessel lock filled with material from the vessel then is pressurized using a suitable gas, such as nitrogen. When a desired target pressure is reached in the container, the container can be opened towards the pressurized volume.

High pressure processes are used for the processing of woody biomass. A possible application is the production of levulinic acid as a precursor to a (bio-)polymer, which is then further processed in another process.

Apparatuses for high pressure processing and applications are described in:
Aktas, Seda; Gevgilili, Halil; Kucuk, Ilknur; Sunol, Aydin; Kalyon, Dilhan M. (2014): Extrusion of poly(ether imide) foams using pressurized CO 2 : Effects of imposition of supercritical conditions and nanosilica modifiers. In Polym Eng Sci 54 (9), pp. 2064-2074. DOI: 10.1002/pen.23753.
Langlotz, Martin; Düngen, Matthias; Koch, Michael (2019): Single screw extrusion with low pressurized blowing agents. In. PROCEEDINGS OF THE EUROPE/AFRICA CONFERENCE DRESDEN 2017 - POLYMER PROCESSING SOCIETY PPS. Dresden, Germany, 27-29 June 2017: Author(s) (AIP Conference Proceedings), p. 70021.
Verreck, Geert; Decorte, Annelies; Li, Hongbo; Tomasko, David; Arien, Albertina; Peeters, Jef et al. (2006): The effect of pressurized carbon dioxide as a plasticizer and foaming agent on the hot melt extrusion process and extrudate properties of pharmaceutical polymers. In The Journal of Supercritical Fluids 38 (3), pp. 383-391. DOI: 10.1016/j.supflu.2005.11.022.
US 3,495,842 and US 5,738,358 A describe pressurized vessels.

Devices operating as vessel locks are described in:
Kiebel, Karl-Heinz (1986): Beschickungs- und Entleerungsvorrichtung in Form einer Vakuum- oder Druck-Rotationsschleuse - European Patent Office - EP 0082947 A1.
Stahl, Werner; Stadler, Reinhold (1984): Systeme zum Ein- oder Austrag von Schüttgütern in oder aus Druckräumen. In Chemie Ingenieur Technik 56 (10), pp. 755-768. DOI: 10.1002/cite.330561005.
US 5,997,220 A, US 5, 997,220 A, DE 10 2013 008 520 A1 and DE 10 2014 113 961 A1 describe vessel locks.

High pressure processes and processing of biomass is described in:
Cha, J.Y; Hanna, M.A (2002): Levulinic acid production based on extrusion and pressurized batch reaction. In: Industrial Crops and Products 16 (2), S. 109-118. DOI: 10.1016/S0926-6690(02)00033-X.
Dautzenberg, Geertje; Gerhardt, Mirko; Kamm, Birgit (2011): Bio based fuels and fuel additives from lignocellulose feedstock via the production of levulinic acid and furfural. In: Holzforschung 65 (4), S. 439-451. DOI: 10.1515/hf.2011.081.
Fitzpatrick, Stephen W. (2006): The Biofine technology: A" bio-refinery" concept based on thermochemical conversion of cellulosic biomass. In: ACS Publications.
Ghorpade, Viswas M.; Hanna, Milford A. (1999): Method and apparatus for production of levulinic acid via reactive extrusion: Google Patents.
Hayes, Daniel J.; Fitzpatrick, Steve; Hayes, Michael H. B.; Ross, Julian R. H. (2006): The biofine process production of levulinic acid, furfural, and formic acid from lignocellulosic feedstocks. In: Biorefineries Industrial Processes and Product 1, S. 139-164.
Rackemann, Darryn W.; Doherty, William O. S. (2011): The conversion of lignocellulosics to levulinic acid. In: Biofuels, Bioprod. Bioref. 5 (2), S. 198-214. DOI: 10.1002/bbb.267.
Raquez, Jean-Marie; Narayan, Ramani; Dubois, Philippe (2008): Recent Advances in Reactive Extrusion Processing of Biodegradable Polymer-Based Compositions. In: Macromol. Mater. Eng. 293 (6), S. 447-470. DOI: 10.1002/mame.200700395.
Thanapal, Siva Sankar; Annamalai, Kalyan; Sweeten, John M.; Gordillo, Gerardo (2012): Fixed bed gasification of dairy biomass with enriched air mixture. In: Applied Energy 97, S. 525-531. DOI: 10.1016/j.apenergy.2011.11.072.

US 5,859,263 A describes a continuous process for the production of levulinic acid from starch in a reactive extrusion process.

DE 349 646 C describes a rotary valve for emptying containers for flowing substances under a pressure other than that prevailing in the emptying chamber.

US 2017/121116 A1 describes a system for discharging dry solids into high pressure environments with a hopper, a feeder device coupled to the hopper, and a discharge device disposed down-stream relative to the feeder device.

US 2009/158664 A1 describes a method for transporting a solid particulate within a gasifier system including discharging solid particulate through an inlet into an opening defined within a rotor, wherein the inlet and the solid particulate are at a first pressure.

Extrusion has also been described for energy compression in biomass such as torrefaction, pyrolysis, combustion, and gasification. The disadvantage of existing processes is that they are usually batch-type processes or continuous processes, but cannot be run with the required process parameters, e.g. pressure, temperature and residence time. The highest possible yield of levulinic acid however can only be expected if the specific parameters of pressure, temperature, acidity, and residence time can be maintained during the process.

### Summary of the invention

The present invention provides a pressure lock, an extruding apparatus for high pressure extrusion, a process for producing biopolymers from biomass and a use of the extruding apparatus for a process for producing biopolymers from biomass allowing continuous operation of a high pressure process according to the independent claims, wherein further embodiments are incorporated in the dependent claims and embodiments described in the following.

According to an aspect of the invention, there is provided a pressure lock for material exchange volumes with a pressure difference, the pressure lock comprising a housing having a cylindrical inner wall, a rotor having a cylindrical outer shape matching gas tight the cylindrical inner wall of the housing and being rotatable in a rotational direction around a rotational axis within the cylindrical inner wall, wherein the housing has a material feeding terminal with a material feeding opening ending in the cylindrical inner wall, a material discharge terminal with a material discharge opening ending in the cylindrical inner wall offset from the material feeding opening, and a first pressure equalizing terminal with a pressure equalizing opening ending in the cylindrical inner wall offset from the material feeding opening and the material discharge opening; wherein the rotor has formed therein a material receiving space having a primary opening ending at the cylindrical outer shape at a longitudinal position with respect to the rotational axis corresponding to the material feeding opening and the material discharge opening, and a secondary opening ending at the cylindrical outer shape at a longitudinal position with respect to the rotational axis corresponding to the first pressure equalizing opening.

Thus, a pressure lock can be provided, which allows a permanent operation by rotating the rotor within the housing and alternating connects the primary opening, which serves for receiving material from a material feeding reservoir and for discharging material to a material discharge volume. As the material feeding reservoir and the material discharge volume may have significant pressure differences, e.g. the material feeding reservoir may be a low pressurized vessel including material and the material discharge volume may be a high pressure extruder, alternating connection results in an alternating pressure difference. This pressure difference, when occurring on the primary opening of the rotor which receives the material, may result in a clogging of the material or turbulent distribution of the material. A pressure equalization over the secondary opening before the primary opening is exposed to the changes of pressure atmosphere significantly reduces the clogging and turbulent distribution of the material during discharging material from the material receiving space of the rotor, as the material receiving space of the rotor can be pressure compensated before being connected to the discharge opening of the housing. This allows a continuous operation of an extruder under high pressure in the entire extruder where a gas pressure inside an extruder can be maintained without slowing down or stopping the continuous operation of the extruder by discontinuous secondary processes even at large pressure differences. The pressure may originate from an external source and may be uniformly distributed throughout the extruder. The pressure may be a gas pressure, which is comparable to the local die pressure in polymer processing.

It should be noted that the rotor within the housing may operate in a combined rotational and alternating forth and back movement along the longitudinal axis, which may result in a combined rotational and oscillating movement of the rotor. Thus the material feeding opening and the material discharge opening may be positioned at different longitudinal positions.

According to an embodiment, the pressure lock operates, so that upon rotation of the rotor within the housing: in a first rotational range the primary opening matches the material feeding opening and the secondary opening faces gas tight a closed portion of the cylindrical wall of the housing, in a subsequent second rotational range the primary opening and the secondary opening both face gas tight a closed portion of the cylindrical wall of the housing, in a subsequent third rotational range the primary opening faces gas tight a closed portion of the cylindrical wall of the housing and the secondary opening matches the first pressure equalizing opening, in a subsequent fourth rotational range the primary opening and the secondary opening both face gas tight a closed portion of the cylindrical wall of the housing, in a subsequent fifth rotational range the primary opening matches the material discharge opening and the secondary opening faces gas tight a closed portion of the cylindrical wall of the housing, and in a subsequent sixth rotational range the primary opening and the secondary opening face gas tight a closed portion of the cylindrical wall of the housing.

Thus a continuous feeding and discharging of the material to and from the material receiving space in the rotor can take place which may underlay a pressure compensation and equalization before discharging. It should be noted that the first to fifth rotational ranges may be consecutive, i.e. without intermediate rotational ranges. The first rotational range may immediately follow the fifth rotational range, unless no second pressure equalization opening is provided, as will be described in detail later.

According to an embodiment, the first pressure equalizing terminal is in a fluid connection with the one of the material feeding terminal and the material discharge terminal, which is under a higher pressure than the other of the material feeding terminal and the material discharge terminal.

Thus, feeding material between different pressure environments can be provided and the material transport on the high pressured side, be it the feeding or the discharging can be carried out without clogging or turbulent distribution of material during material transport.

According to an embodiment, the first pressure equalizing terminal is in a fluid connection with the material discharge terminal.

Thus, feeding material from a low pressure environment to a high pressure environment can be provided and discharging material at the high pressure side can be carried out without clogging or turbulent distribution of material during discharge.

According to an embodiment the rotor with respect to an axial direction along the rotational axis is fixed with respect to the housing and is rotatable in a rotational direction with respect to the housing, wherein the material feeding opening and the material discharge opening with respect to the rotational axis are substantially opposite to each other.

Thus, a high speed processing during introducing material to a high-pressurized environment can be achieved, in particular if the movement of the rotor is limited to a rotational movement around the rotational axis only, i.e. without axial movement along the rotational axis. Further, the gas tight sealing in said axial direction can be improved as without axial movement, also no axial scratches can occur on the cylindrical surfaces of the housing or of the rotor.

According to an embodiment, the material feeding opening and the material discharge opening with respect to the rotational axis are at the at the same axial position with respect to the rotational axis, and offset in rotational direction, in particular offset in rotational direction by 180°.

Thus, in particular when aligning the material feeding opening upwards, and the material discharge opening downwards, both, the material feeding and the material discharging can be carried out or at least be supported by gravity.

According to an embodiment, the secondary opening with respect to the axial direction is offset from the primary opening.

Thus, it can be avoided that the secondary opening unintentionally matches with the material feeding opening or the material discharge opening, which may result in a clogging of the openings. In particular, if the secondary opening is of a smaller diameter than the material feeding opening or the material discharge opening, residual material may clog the secondary opening, i.e. the pressure equalizing or pressure compensating opening. Separating the secondary opening from the material feeding opening and from the material discharge opening may avoid that.

According to an embodiment, the secondary opening with respect to the rotational direction is offset from the primary opening.

Thus, also for a sole rotational movement of the rotor, it can be avoided that the secondary opening unintentionally matches with the material feeding opening or the material discharge opening, which may result in a clogging of the openings.

According to an embodiment, the secondary opening with respect to the rotational direction is offset from the primary opening by an angle of approximately 45°.

Thus, an optimum geometry can be achieved and a spatial collision of the material feeding terminal and the material discharge terminal with the pressure equalizing terminal at the housing can be avoided.

According to an embodiment, the housing further has a second pressure equalizing terminal with a pressure equalizing opening ending in the cylindrical inner wall offset from the material feeding opening and the material discharge opening; wherein the secondary opening ends at the cylindrical outer shape at an axial position with respect to the rotational axis corresponding to the first pressure equalizing opening and the second pressure equalizing opening.

Thus, the pressure equalization or pressure compensation can be carried out not only after the feeding and before the discharging, which avoids clogging and turbulent material distribution upon discharging, but also in the further rotational direction, after the discharging and before the further feeding, which avoids clogging and turbulent material distribution upon feeding. Thus, the pressure equalization may be carried out two times with every full rotation. Pressurization happens just before the material can fall down into the processing system. After that, depressurization may be carried out before completing a full rotation to avoid clogging and turbulent material distribution.

According to an embodiment, the pressure lock operates, so that upon rotation of the rotor within the housing: in a first rotational range the primary opening matches the material feeding opening and the secondary opening faces gas tight a closed portion of the cylindrical wall of the housing, in a subsequent second rotational range the primary opening and the secondary opening both face gas tight a closed portion of the cylindrical wall of the housing, in a subsequent third rotational range the primary opening faces gas tight a closed portion of the cylindrical wall of the housing and the secondary opening matches the first pressure equalizing opening, in a subsequent fourth rotational range the primary opening and the secondary opening both face gas tight a closed portion of the cylindrical wall of the housing, in a subsequent fifth rotational range the primary opening matches the material discharge opening and the secondary opening faces gas tight a closed portion of the cylindrical wall of the housing, in a sixth rotational range the primary opening and the secondary opening both face gas tight a closed portion of the cylindrical wall of the housing, in a subsequent seventh rotational range the primary opening faces gas tight a closed portion of the cylindrical wall of the housing and the secondary opening matches the second pressure equalizing opening, and in a subsequent eighth rotational range the primary opening and the secondary opening both face gas tight a closed portion of the cylindrical wall of the housing.

Thus, a continuous feeding and discharging of the material to and from the material receiving space in the rotor can take place, which may underlay a pressure compensation and equalization before discharging and before feeding. It should be noted that the first to the eighth rotational ranges may be consecutive, i.e. without any further intermediate rotational ranges. The first rotational range may immediately follow the eighth rotational range, unless no further pressure equalization opening is provided.

According to an embodiment, the second pressure equalizing terminal is in a fluid connection with the one of the material feeding terminal and the material discharge terminal, which is under a lower pressure than the other of the material feeding terminal and the material discharge terminal.

Thus, the feeding between different pressure environments can be provided and the material transport on the low pressurized side, be it the feeding or the discharging can be carried out without clogging or turbulent distribution of material during material transport.

According to an embodiment, the second pressure equalizing terminal is in a fluid connection with the material feeding terminal.

Thus, the feeding from a high-pressure environment to a low-pressure environment can be provided and the discharge at the low-pressure side can be carried out without clogging or turbulent distribution of material during discharge.

According to a further aspect of the invention, there is provided an extruding apparatus for high pressure extrusion processing, the apparatus comprising: at least one extruder screw having a driving rod to be coupled to a drive; a housing having accommodated in a housing interior the at least one extruder screw, the housing having a lead through opening for the driving rod, a feeding port for feeding material to the housing interior, a material outlet nozzle for letting out extruded material, and a pressure port for pressurizing the housing interior; a high pressure sealing at the lead through opening for the driving rod; and a pressure lock as described above at the feeding port for feeding material to the housing interior maintaining the pressure within the housing interior during material feeding.

Thus, an extruding apparatus can be provided which is capable of carrying out an extrusion process, which is entirely under pressure and the generation of a completely continuous process from the starting material to the finished polymer with the aid of an extruder is achieved, e.g. the separation of levulinic acid as polymeric starting material may be carried out with the aid of a pressurized extruder. The pressure can create a natural boundary that prevents the formation of small molecules, and thus de-polymerization and by-products. The high number of by-products may be reduced and thus low yield of e.g. levulinic acid as well as possible de-polymerization in the continuous process is avoided, since necessary process parameters, such as pressure, cannot be implemented in the process. The drive side of the extruder at the lead through of the driving rod of the extruder screw is sealed e.g. by a plate which uses special high pressure hydraulic seals. These seals can withstand pressures up to 300 bar and are highly wear resistant. Such a seal may be made at each lead through of a drive or even a rod stud. As a pressure lock a pressure lock as described above is used.

The pressure within the extruder housing may be applied through the pressure port to which a pressure source may be coupled. The pressure may be controlled, in particular feedback controlled so that the target pressure may be maintained. The pressure port may have different endings in the housing, so that the pressure applied to the pressure port is spatially distributed. The different endings may underlay a different pressure control, so that different pressures may be applied to different regions of the extruder housing. Thus, defined processing zones with pressure gradients can be created. E.g., at the beginning of the process, a zone can be defined so that all material fed in is crushed to a desired size. Other possible functions comprise the creation of a pressure and reaction zone.

According to an embodiment, the pressure lock is a pressure lock as describe above in detail, wherein the material discharge terminal is connected gas tight to the material feeding port and which pressure lock is adapted for feeding material into the housing interior, wherein the pressure lock is adapted to separate an atmospheric pressure from a process pressure within the housing interior.

Thus, the extruding apparatus may gain from the benefits of the pressure lock described above. The effects and advantages of the pressure lock as described above apply mutatis mutandis also to the extruding apparatus. In particular, the feeding of material without clogging and without turbulent material distribution upon the discharging phase can be carried out while maintaining the pressure level within the housing of the extruding apparatus. The material discharge opening of the pressure lock may be disposed immediately above a feeding area of the extruding screw(s) so that no further feeding facilities may be required. The avoided clogging and turbulences by pressure equalization may lead to a targeted feeding of the material out of the material receiving space of the rotor to the extruding screw(s).

According to an embodiment, the extruding apparatus comprises a first extruder screw and a second extruder screw, each having a driving rod, thus forming a twin extruder, wherein the housing has a first and a second lead through opening for the driving rod of the first and the second extruder screw, each lead through having a separate high pressure sealing at the lead through opening for the respective driving rod.

Thus, a reliably operating extruder type can be provided. Twin extruders may be provided as different sized extruders allowing the process to be easily scaled. The sealing aspects mutatis mutandis apply for the second extruder screw.

According to an embodiment, the extruding apparatus further comprises a pressure source being connected to the pressure port, wherein the pressure source is a gas pressure source.

Thus a uniform and reliable pressure can be maintained in the housing. The pressure source may be a reservoir or may be a generator for generating a pressure or a combination thereof. The pressure source may provide introducing and controlling the gas pressure, which may also be used as a foaming agent to be dispensed with in a foaming process and or to produce (additional) gas pressure locally.

According to an embodiment, the pressure source is a nitrogen gas pressure source, in particular a nitrogen gas pressure source being adapted for applying at least 25 bar to the housing interior.

Thus, nitrogen may create an inert atmosphere that prevents oxidation of e.g. cellulose structure and thus degradation to ash. The process pressure helps to increase the yield of certain molecule sizes and thus chemicals e.g. levulinic acid. The pressure source may include a gas separator for separating nitrogen from environmental air.

According to an embodiment, the pressure source and the pressure port are adapted for applying gas pressure globally inside the whole system, compared to local pressure with polymer pressure build-up.

Thus, a pressure distribution can be set within the housing depending on the need for pressure during the extrusion phases.

According to an embodiment, the material outlet nozzle for letting out extruded material is adapted for being modified with respect to the outlet geometry of the material outlet nozzle, in particular with respect to the fluid flow resistance of the outlet nozzle geometry, in particular with respect to the outlet cross-section of the material outlet nozzle.

Thus, the flow resistance of the nozzle can be adapted according to the viscosity of the material to be extruded, and a sealing can be achieved at the outlet side of the extruding apparatus. This may be helpful when melt-like or highly viscous media are to be processed. If a polymer is not able to provide a seal due to its structure and viscosity, the nozzle diameter can still be further reduced and flow resistance increased accordingly. This increased flow resistance results in a better seal.

According to an embodiment, the extruding apparatus comprises a second pressure lock downstream the material outlet nozzle for letting out material from the housing interior, wherein the second pressure lock is adapted for maintaining the pressure within the housing interior during material outlet.

Thus, particularly if no polymer is processed, but solids, another pressure lock can be placed downstream the material outlet nozzle, which provides a second sealing point. A second pressure lock in combination with an outlet geometry of the material outlet nozzle, which can be modified enable to process a large number of possible materials by one and the same extruder using a pressurized atmosphere. Depending on the material and the process, a second pressure lock is needed to discharge the product from the extruder.

It should be noted that alternatively a pressure reducer can be provided as a second pressure lock, which may be located behind a condenser.

According to an embodiment, the extruding apparatus further comprises a heater being adapted for heating the housing interior, in particular for heating the housing interior with a heating gradient along a longitudinal extension of the housing.

Thus, at the extruding process can be carried out by modifying the temperature at all and also at different locations of the extruding apparatus individually.

According to an embodiment, the extruding apparatus further comprises a separator unit downstream the material outlet nozzle for letting out material from the housing interior, in particular a cyclone separator being adapted for separating non-gaseous particles from gaseous particles, wherein the housing has a non-gaseous particle port for feeding back the non-gaseous particles from the separator unit to the housing interior.

Thus, after the material has left the extruder through the material outlet nozzle, it may be e.g. accelerated by a gas pump and the remaining, not yet dissolved particles are separated from the gas. In particular non-gaseous particles can be separated from gaseous particles, and non-gaseous particles can be fed back to the extruding apparatus. Separation can be carried out e.g. by a cyclone separator. The solids can be fed back into the twin screw extruder where they are further disintegrated. This cycle provides a high yield of e.g. levulinic acid, as almost all biomass initially provided to the extruding apparatus can be gasified if desired.

According to an embodiment, the extruding apparatus further comprises a steam condenser downstream the separator unit, the steam condenser being adapted for condensing the gaseous particles.

Thus, by condensation the pressure can be reduced downstream.

According to an embodiment, the extruding apparatus further comprises a pressure reducer downstream the condenser.

Thus, at the pressure reducer, the liquid condensate may seal off the process so that, together with the optional downstream pressure lock, a continuously conveying but also pressurized extruding apparatus operating as a reactor is created. The pressure reducer may also operate as the second pressure lock, and may also equalize the process pressure again.

According to an embodiment, the extruding apparatus further comprises a distiller downstream the condenser, in particular a fractional distiller and a subsequent vacuum distiller.

Thus, the mixture of substances may be separated by fractional distillation and e.g. levulinic acid may be separated. Levulinic acid may then be further processed to a biopolyester.

According to an embodiment, the extruding apparatus further comprises a polymerization unit downstream the distiller.

Thus, the output of the extruding apparatus can be directly fed to a polymerization unit or the output can be further converted into other polymerizable compounds.

An aspect of the invention refers to the use of the extruding apparatus as described above for high pressure extrusion processing of biomass, wherein the biomass is preferably woody biomass.

Thus, a biomass, in particular a woody biomass can be processed. By reactive extrusion, a processing of woody biomass can be carried out e.g. using temperature, acid and pressure control.

According to an aspect of the invention, there is provided a process for producing a composition from biomass, particularly a chemical compound from biomass, particularly a biopolymer from biomass using the extruding apparatus as described above, the process comprising continuously feeding through the pressure lock biomass into the housing interior of the extruding apparatus and processing of the biomass by at least one of a temperature application to the biomass in the housing interior of the extruding apparatus, an acid application to the biomass within the housing interior of the extruding apparatus and a pressure change to the biomass within the housing interior of the extruding apparatus.

In particular, there is provided a process for producing a composition from biomass, particularly a chemical compound from biomass, particularly a biopolymer from biomass using the extruding apparatus as describe above, the process comprising continuously feeding through the pressure lock biomass into the housing interior of the extruding apparatus and processing of the biomass by at least one of a temperature application to the biomass in the housing interior of the extruding apparatus, an acid application to the biomass within the housing interior of the extruding apparatus and a pressure change to the biomass within the housing interior of the extruding apparatus, thus dissolving gas from the biomass; continuously letting out the dissolved gas from the material outlet nozzle, wherein optionally the process further comprises separating non-gaseous particles from gaseous particles and feeding back the non-gaseous particles to the housing interior of the extruding apparatus.

Thus, a continuous process of high pressure processing can be carried out with biomass, in particular woody biomass. For example, sawdust may be conveyed forward in the extruder, wherein the high pressure processing of the wood structures consistently dissolve to gas.

According to an embodiment, the process further comprises distillation of the gaseous particles, in particular fractional distillation of the gaseous particles for obtaining levulinic acid and subsequent vacuum distillation thereof, and optionally subsequent transformation of the produced levulinic acid to monomers, and further optionally subsequent production of biodegradable polyesters from the monomers, in particular using at least one of a chemical and an enzymatic polymerization process.

Thus, a further separation and extraction can be managed, in particular, any means for a separation and/or extraction mechanism by fractional distillation to obtain levulinic acid from a mixture of biological acids can be used.

According to an embodiment, the biomass is woody biomass, preferably selected from the group consisting of sawdust, shredded residual wood, wood residues and a composition thereof, wherein the woody biomass in particular has a moisture content of more than 30%, in particular of more than 50%.

Thus, the process may start with the raw material, i.e. the biomass, which may be a wet sawdust. Alternative raw materials are shredded residual wood or wood residues of any kind. Wet sawdust has a moisture content of about 50%, which is not only positive for the later process, but also makes prior drying unnecessary for later use of the extruded wood mass as e.g. wood pellets. This may save effort and energy.

It should be understood that the described embodiments can also be combined and that a combination thereof may have a synergetic effect which may extend over the sum of the single effects.

The accompanying drawings are intended for providing a further understanding of embodiments of the present invention. The accompanying drawings illustrate embodiments and, in connection with the description, serve to explain concepts of the present invention.

### Brief description of the figures

The invention will be described in detail referring to the following figures, in which:
- Figure 1: illustrates a perspective view of a pressure lock according to an exemplary embodiment of the invention.
- Figure 2: illustrates a perspective view of a rotor and terminals at the housing without the housing as such according to an exemplary embodiment of the invention.
- Figure 3: illustrates a side view of a rotor and material discharge terminal without the housing as such according to an exemplary embodiment of the invention.
- Figure 4: illustrates a cross-sectional view of a pressure lock orthogonal to a rotational axis according to an exemplary embodiment of the invention.
- Figure 5A-H: illustrate cross-sectional views a pressure lock orthogonal to a rotational axis in different phases of rotation according to an exemplary embodiment of the invention.
- Figure 6: illustrates a schematic build-up of an extruding apparatus according to an exemplary embodiment of the invention.
- Figure 7: illustrates a schematic build-up of an extruding apparatus with subsequent processing units according to an exemplary embodiment of the invention.
- Figure 8: illustrates a schematic flow chart of a process according to an exemplary embodiment of the invention.

The invention will be described in detail, wherein similar or same components have the same reference numbers. If for a particular figure a component is not described, a description applies which was made with respect to other figures.

### Detailed description of exemplary embodiments

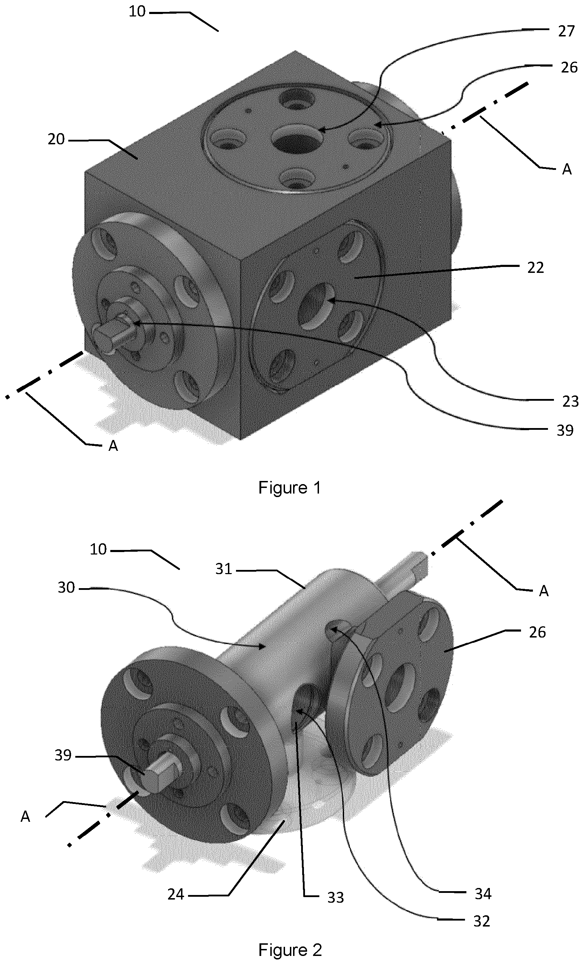

Figure 1 illustrates a perspective view of a pressure lock 10 according to an exemplary embodiment of the invention. The pressure lock 10 comprises a housing 20 and a rotor 30, which resides within the housing 20 and is not visible in Figure 1. The rotor 30 can be driven by a rotor drive coupling 39 providing a rotational movement of the rotor 30 around its rotational axis A. The rotor 30 has a material receiving space 32 having a primary opening 33 for feeding and discharging material to and from the material receiving space 32, respectively. The primary opening 33 for feeding material is in an axial and rotational position of the rotor 30 so that it matches the material feeding opening 23 of the housing 20, in Figure 1 top. The material feeding terminal 22 serves for mounting the housing 20 in a gas tight manner to a material reservoir (not shown). The material receiving space 32 further has secondary opening 34 apart from the primary opening 33 for ventilation. The distinct secondary opening 34 allows ventilation though a different conduit than the material feeding and discharging primary opening 33. For pressure equalization, the secondary opening 34 matches the pressure equalizing opening 27 in the housing 20. The pressure equalizing terminal 26 serves for connecting the pressure equalizing opening 27 in a gas tight manner to a pressured volume (not illustrated in Figure 1).

Figure 2 illustrates a perspective view of a rotor 30 and terminal 26 at the housing 20 without the housing 20 as such according to an exemplary embodiment of the invention.

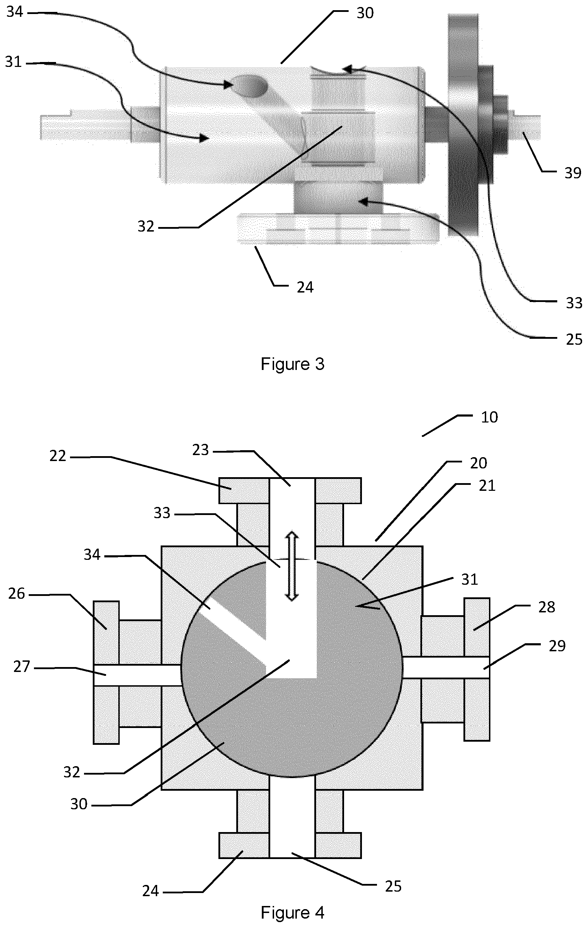

Figure 3 illustrates a side view of a rotor 30 and material discharge terminal 24 without the housing 20 as such according to an exemplary embodiment of the invention. In order to realize the issue of high pressures as well as easy maintenance in a pressure lock 10, ventilation adapters in form of pressure equalizing terminals 26 (not shown) have been realized which were integrated into an outer housing 20 of a pressure lock 10. The pressure equalizing terminals 26, 28 (not shown) seal a pressure vessel for both pressurization and depressurization. The pressure lock 10 is provided with a cylindrical rotor 30 with a material receiving space 32 formed therein provided with two holes, a primary opening 33 for receiving and discharging material to be fed and a secondary opening 34 for pressure equalization or pressure compensation. The primary opening 33 is used to convey material from the material feeding terminal/opening 22/23 (not shown) to a material discharge terminal/opening 24/25 by rotating it around a rotational axis. The secondary opening 34 in this embodiment is drilled diagonally to the upper plane, where it extends into the material receiving space 32. Its purpose is to act as a pressure vent hole for the material receiving space 32. It should be noted that the material receiving space 32 can be formed by a drilling process and the material receiving space 32 may have the same diameter as the primary opening 33.

For the pressure equalization or venting, two separate adapters or terminals 26, 28 (not shown) are required on the housing 20. The terminals 26, 28 are located on both sides of the pressure lock housing 20 and are used only for pressurization and pressure relief. In the illustrated embodiments, the material feeding opening 23 is located at the top and the material discharge opening 25 at the bottom of the housing 20.

Material falls into the material feeding opening 23 and remains there until the primary opening 33 of the rotor 30 is aligned upward. After a 180° rotation, the primary opening 33 of the rotor 30 is aligned downward and the material received in the material receiving space 32 of the rotor 30 can fall/be discharged through the material discharge opening 25 of the material discharge terminal 24.

If the material feeding opening 23 is aligned vertical and radial to the cylinder, the material may be fed by gravity. In order to avoid pressure loss in the pressurized system, any connection between the material discharge terminal/opening 24/25 and the pressure equalizing terminal/opening 26/27 as a pressure vent at the same time should be prevented, and any connection between the material feeding terminal/opening 22/23 and the pressure equalizing terminal/opening 26/27 should be prevented. In other words, gas should not be able to flow from the high pressure housing interior 71 to the outside of the pressure lock 10 during material feeding. This would create a small window where the system would quickly lose pressure. To avoid this, there must be a time gap between the pressure equalization/depressurization and the refilling of the material receiving space 32. During this gap period both, the primary opening 33 and the secondary opening 34 must be completely sealed to prevent this pressure loss. The position of the secondary opening 34 for this purpose may be offset in the rotational direction by 45° from the primary opening 33, relative to the cylinder. This configuration allows a smooth transition between pressurization and depressurization.

The terminals 22, 24, 26, 28 establish a gas tight connection between the pressure lock 10 and the components connected thereto. In some cases the venting is to the atmospheric pressure, so that no special connection is required.

A 45° offset between the primary and the secondary opening 33, 34 enables a smooth transition between the different stages. Figure 2 shows a crucial point of the pressurization. The secondary opening 34 begins to fade into the pressure equalizing terminal/opening 26/27 while the material filled material receiving space 32 with the primary opening 33 is exactly between the upper material feeding opening 23 (not shown as housing is removed in the Figure) and lower material discharge opening 25 (not shown as housing is removed in the Figure), i.e. lateral. Now the entire material receiving space 32 is pressurized as the rotor 30 continues to rotate onward, clockwise in Figure 3. Shortly thereafter the primary opening 33 reaches the lower material discharge opening 25 and the material can fall down, as there is no longer a pressure difference. If no pressure equalization takes place, pressurization would occur very quickly and toward the primary opening 33, so that material particles would be scattered and turbulently distributed and potentially clogged in the primary opening 33. The same phenomenon can be expected on the opposite side of the pressure lock, during venting. In order to avoid swirling of the bulk material, the material receiving space 32 must be vented before the primary opening 33 reaches the top material feeding opening 23. Otherwise the pressure release through the primary opening 33 swirls the material upward. The depressurization is done according to the same principle as the pressurization.

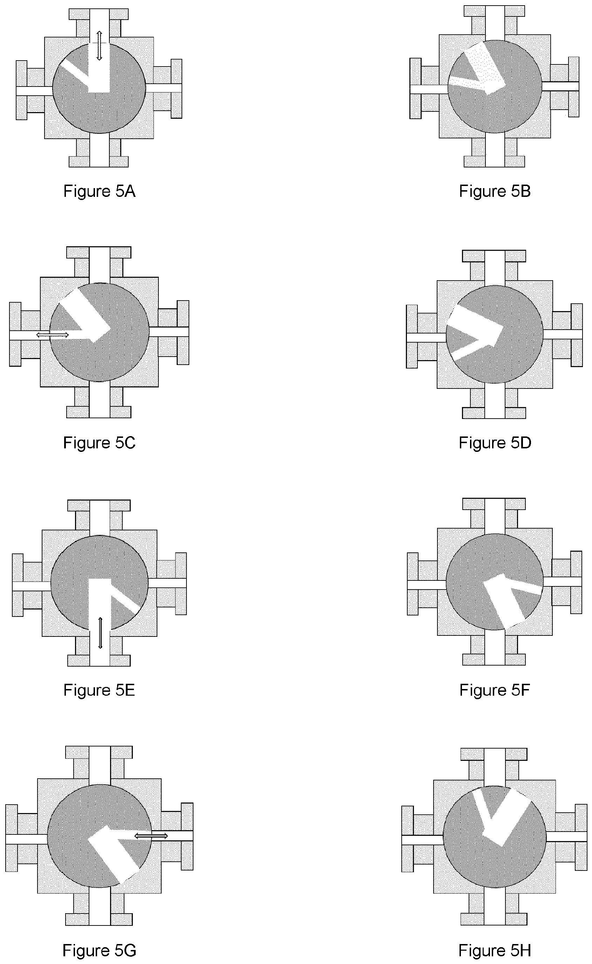

Figure 4 illustrates a cross-sectional view of a pressure lock 10 orthogonal to a rotational axis according to an exemplary embodiment of the invention. The pressure lock 10 comprises a housing 20 having a cylindrical inner wall 21. The housing 20 in the illustrated embodiment has a material feeding terminal 22 with a material feeding opening 23 on the top side, and on the bottom side a material discharge terminal 24 with a material discharge opening 25. For pressure equalization there is provided a first pressure equalizing terminal 26 having a first pressure equalizing opening 27. Optionally the pressure lock 10 may have a second pressure equalizing terminal 28 with a second pressure equalizing opening 29. A rotor 30 with a cylindrical outer shape 31 rotates in a gas tight manner in the cylindrical inner wall 21 of the housing 20. The rotor has formed therein a material receiving space 32, which has a communication to the cylindrical outer shape surface 31 of the rotor 30 through a primary opening 33 for material charge and discharge and a secondary/pressure equalizing opening 34 for pressure equalization or pressure compensation. It should be noted that the pressure lock 10 as illustrates in Figure 4 may also be provided without the second pressure equalizing terminal 28 and without the second pressure equalizing opening 29, e.g. in case the pressure equalization is crucial only in one direction, e.g. for material discharge. Upon rotation, the rotor alternating charges and discharges material, and drops the material in small portions into a pressured volume connected to the bottom material discharge terminal 24. This device has the advantage that it operates continuously and does not require any control technology. The concept of the pressure equalization mechanism and the sealing technology will be described with respect to Figures 5A to 5H.

For Figures 5A to 5G it should be noted that the pressure equalizing openings 27, 29 at the cylindrical inner wall 21 of the housing 20 are offset in axial direction, i.e. in the direction of the rotational axis, from the material feeding opening 23 and material discharge opening 25. This means, the pressure equalizing openings 27, 29 at the cylindrical inner wall 21 of the housing 20 do not match to/do not align with the primary opening 33 of the rotor 30 in any rotational orientation of the rotor 30, and the material feeding opening 23 and the material discharge opening 25 do not match to/do not align with the secondary opening 34 of the rotor 30 in any rotational orientation of the rotor 30. However, for illustrative purposes, in Figures 5A to 5G the pressure equalizing openings 27, 29 at the cylindrical inner wall 21 of the housing 20 and the material feeding opening 23 and the material discharge opening 25 are illustrated in the same cross-sectional plane.

Figure 5A to Figure 5F illustrate the operation of the pressure lock 10 during subsequent phases of rotation of the rotor 30, the pressure lock 10 having only one pressure equalizing opening 27 (the second pressure equalizing terminal 28 and the second pressure equalizing opening 29 shown in Figs. 5A-5F are to be ignored). The subsequent phases of the rotational operation are as follows: Upon rotation of the rotor 30 within the housing 20: In a first rotational range illustrated in Figure 5A the primary opening 33 matches the material feeding opening 23 and the secondary opening 34 faces gas tight a closed portion of the cylindrical inner wall 21 of the housing 20. In a subsequent second rotational range illustrated in Figure 5B the primary opening 33 and the secondary opening 34 both face gas tight a closed portion of the cylindrical inner wall 21 of the housing 20. In a subsequent third rotational range illustrated in Figure 5C the primary opening 33 faces gas tight a closed portion of the cylindrical inner wall 21 of the housing 20 and the secondary opening 34 matches the first pressure equalizing opening 27. In a subsequent fourth rotational range illustrated in Figure 5D the primary opening 33 and the secondary opening 34 both face gas tight a closed portion of the cylindrical inner wall 21 of the housing 20. In a subsequent fifth rotational range illustrated in Figure 5E the primary opening 33 matches the material discharge opening 25 and the secondary opening 34 faces gas tight a closed portion of the cylindrical inner wall 21 of the housing 20. In a subsequent sixth rotational range illustrated in Figure 5F the primary opening 33 and the secondary opening 34 both face gas tight a closed portion of the cylindrical inner wall 21 of the housing 20. Then a full revolution is done and after the sixth phase illustrated in Figure 5F the rotor succeeds with the first rotational phase illustrates in Figure 5A as described above. It should be kept in mind that for the afore described embodiment, the second pressure equalizing terminal 28 and the second pressure equalizing opening 29 are to be ignored, so that for the afore described embodiment Figures 5G and 5H are without relevance.

Figure 5A to Figure 5H illustrate the operation of the pressure lock 10 during subsequent phases of rotation of the rotor 30, the pressure lock 10 having a first pressure equalizing opening 27 and second pressure equalizing opening 29. The subsequent phases of the rotational operation are as follows: Upon rotation of the rotor 30 within the housing 20: In a first rotational range illustrated in Figure 5A the primary opening 33 matches the material feeding opening 23 and the secondary opening 34 faces gas tight a closed portion of the cylindrical inner wall 21 of the housing 20. In a subsequent second rotational range illustrated in Figure 5B the primary opening 33 and the secondary opening 34 both face gas tight a closed portion of the cylindrical inner wall 21 of the housing 20. In a subsequent third rotational range illustrated in Figure 5C the primary opening 33 faces gas tight a closed portion of the cylindrical inner wall 21 of the housing 20 and the secondary opening 34 matches the first pressure equalizing opening 27. In a subsequent fourth rotational range illustrated in Figure 5D the primary opening 33 and the secondary opening 34 both face gas tight a closed portion of the cylindrical inner wall 21 of the housing 20.In a subsequent fifth rotational range illustrated in Figure 5E the primary opening 33 matches the material discharge opening 25 and the secondary opening 34 faces gas tight a closed portion of the cylindrical inner wall 21 of the housing 20. In a sixth rotational range illustrated in Figure 5F the primary opening 33 and the secondary opening 34 both face gas tight a closed portion of the cylindrical inner wall 21 of the housing 20. In a subsequent seventh rotational range illustrated in Figure 5G the primary opening 33 faces gas tight a closed portion of the cylindrical inner wall 21 of the housing 20 and the secondary opening 34 matches the second pressure equalizing opening 29. In a subsequent eighth rotational range illustrated in Figure 5H the primary opening 33 and the secondary opening 34 both face gas tight a closed portion of the cylindrical inner wall 21 of the housing 20. Then a full revolution is done and after the eighth phase illustrated in Figure 5H the rotor succeeds with the first rotational phase illustrated in Figure 5A as described above.

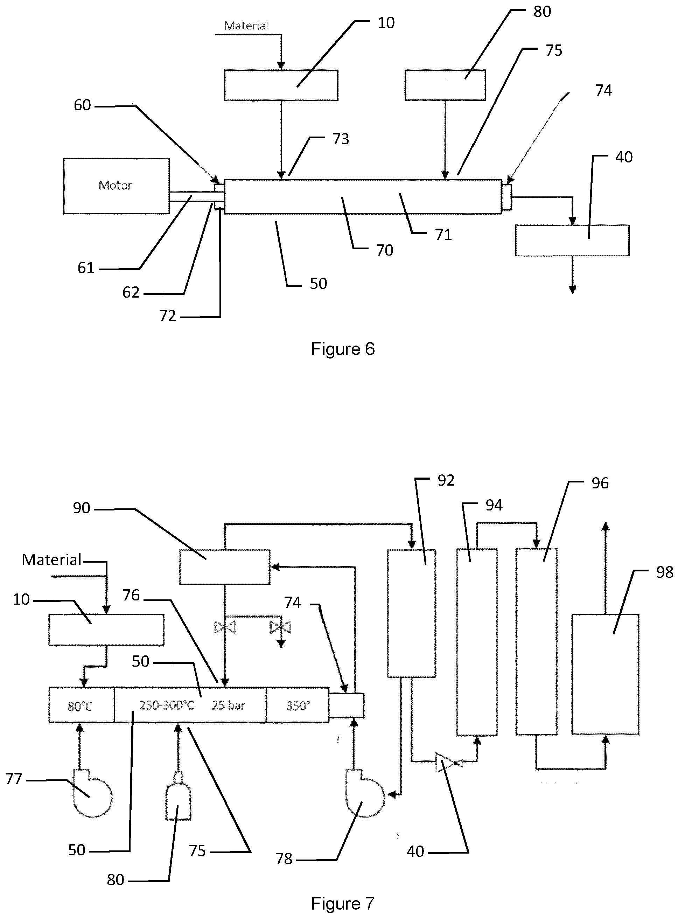

Figure 6 illustrates a schematic build-up of an extruding apparatus according to an exemplary embodiment of the invention. The basic pressurized extrusion process, which is carried out with an extruding apparatus 50 as illustrated in Figure 6 can be technically described as follows:
A drive side of the extruder, i.e. a drive rod 61 of the extruder screw 60 is sealed by a high pressure sealing 62 (not shown) over a lead though opening 72 for the extruder drive rod 61 to the housing interior 71 of housing 70, which uses special high pressure hydraulic seals. These seals can withstand pressures up to 300 bar and are highly wear resistant. Such a seal is provided at the rear end of the extruder. If a twin extruder is used, these high-pressure seals enclose both extruder screws individually and separately. This provides a complete seal of both extruder screws to the drive side.

A further sealing concept is required on the feeding side for feeding material to the extruding apparatus 50 through a material feeding port 73. For feeding material continuously, a pressure lock 10 as described above with respect to Figure 1 to Figure 5H is used.

In addition, a sealing concept is required for the output side, i.e. toward the material outlet nozzle 74. Depending on the material and the process, a second pressure lock 40 is required to discharge the product from the extruder. If processed material is a polymer or a similar viscous substance, the material can act as a seal even at high pressures. As a viscous material even when flowing through the material outlet nozzle 74 may act as a seal, as the flow velocity is low compared to the provided pressure. Thus, the gas pressure caused by the melted polymer cannot escape through the material outlet nozzle 74. The sealing on the output side by viscous material in the material outlet nozzle 74 is suitable if molten or highly viscous media are to be processed. If a viscosity of a polymer is not capable of ensuring a seal, the nozzle diameter can still be reduced. This increases the flow resistance and provides better seal. If no polymer but solids are being processed, another pressure lock 40 can be placed downstream the material outlet nozzle 74 to provide a second sealing barrier. With these two options, the process is able to process a large number of possible materials by one and the same extruder using a pressurized atmosphere, which is provided by a pressure source 80 through a pressure port 75.

Figure 7 illustrates a schematic build-up of an extruding apparatus with subsequent processing units according to an exemplary embodiment of the invention. With respect to the description of the extruding apparatus 50 it is referred to Figure 6. In addition, the extruding apparatus 50 may be provided with a feeding 77 of (sulfuric)acid, which may support in particular the biomass processing. Further, the extruding apparatus 50 may be provided with a heating capability, which may selectively heat the extruding apparatus housing interior 71.

As can be seen from Figure 7, downstream the material outlet nozzle 74, the extruding apparatus 50 may comprise a gas blower 78 and a separator unit 90, e.g. a cyclone separator, which may separate gaseous and non-gaseous particles. Gaseous particles may then be fed to a steam condenser 92 arranged further downstream. Non-gaseous particles may be fed back to the extruding apparatus 50 through a feedback port 76. The non-gaseous particles before feeding back to the housing interior 71, may be further separated, so that e.g. ashes can be separated and not be fed back to the housing interior 71. Downstream the condenser 92, a pressure reducer 40 may be provided, which may also operate as a pressure lock 40. Further downstream the condenser 92 and the pressure reducer 40, the apparatus may comprise a distiller 94, 96, which may be at least one of a fractional distiller 94 and a vacuum distiller 96. Further downstream, a polymerization unit 98 may be provided.

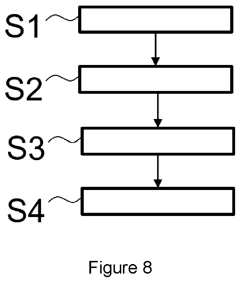

Figure 8 illustrates a schematic flow chart of a process according to an exemplary embodiment of the invention. The process may comprise continuously feeding through the pressure lock 10 biomass into the housing interior 71 of the extruding apparatus 50 and processing S1 of biomass by at least one of a temperature application to the biomass in the housing interior 71 of the extruding apparatus 50, an acid application, in particular a sulfonic acid application through an acid feeding port 77 to the biomass within the housing interior 71 of the extruding apparatus 50 and a pressure change to the biomass within the housing interior 71 of the extruding apparatus 50, thus dissolving gas from the biomass. Likewise, the dissolved gas is led out continuously from the material outlet nozzle 74. The process may optionally include separating non-gaseous particles from gaseous particles and feeding back the non-gaseous particles to the housing interior 71 of the extruding apparatus 50 through a feedback port 76. Based on the dissolved gas, the process may further comprise distillation of the gaseous particles S2 in a distilling facility 94, 96 as described above, in particular fractional distillation of the gaseous particles for obtaining levulinic acid and subsequent vacuum distillation thereof. Afterwards and optionally, a transformation S3 of the produced levulinic acid to monomers like succinic acid or butandiol can be carried out, and further optionally a subsequent production S4 of biodegradable polyesters from the monomers, in particular using at least one of a chemical and an enzymatic polymerization process may take place.

As the extruding apparatus 50 allows a high pressure operation, the high pressure may largely prevent de-polymerization and uncontrolled degradation of e.g. chemical wood structure to undesired by-products. The extruding apparatus 50 and the process carried out by using the extruding apparatus 50 allow transferring and adapting the parameters of batch processes to a continuous process, so that maintenance efforts and energy costs can be minimized. By focusing on woody biomass, raw material costs can be massively reduced. The developed process, which may be carried out by the apparatus illustrated in Figures 6 and 7, can be carried out on biomass, in particular woody biomass. This process along the procedure described with respect to Figure 8 can be described in four process steps.

At first, a reactive extrusion S1 is carried out. Woody biomass is processed by using temperature, acid and/or pressure. Then, a separation/extraction S2 is carried out, where a separation/extraction mechanism by fractional distillation is used to obtain levulinic acid from a mixture of biological acids. Afterwards, chemical conversion or chemical transformation S3 is carried out of the produced levulinic acid to the required monomers. Then a polymerization of biodegradable polyesters using chemical or enzymatic polymerization processes S4 is carried out.

The reactive extrusion S1 will now be described in more detail. This includes generation of a completely continuous process from the starting material to the finished polymer supported by a continuously operating extruding apparatus 50. The cleavage of the levulinic acid as the polymeric starting material takes place with a pressurized extruder. The process starts with the raw material, which may be wet sawdust. Other possible materials are shredded residual wood or wood residues of any kind. The wet sawdust has a moisture content of about 50%, which is not only positive for the later process, but also makes prior drying unnecessary for later use of the extruded wood mass as e.g. wood pellets. The sawdust is fed to the pressurized extruding apparatus 50 through an above described pressure lock 10 into e.g. a twin-screw extruder. The pressure lock 10 provides a continuous seal of the material inlet, as described above. The extruding apparatus 50 may be pressurized with nitrogen from a pressure source 80 e.g. at a process pressure of 25 bar, which is provided by the pressure source 80 through a pressure port 75. The nitrogen also creates an inert atmosphere that prevents oxidation of the cellulose structure and thus degradation to ash. As described previously, the process pressure helps to increase the yield of certain molecule sizes and thus chemicals - e.g. levulinic acid. Thus, as the sawdust is conveyed forward in the twin-screw extruder, the wood structures consistently dissolve to gas. This degradation process is aided by both temperature and introduced dilute sulfuric acid through acid feeding port/acid feeding source 77. At the pressure reducer 40, the liquid condensate seals off the process so that, the pressure reducer 40 operating as a second pressure lock 40 or together with a second pressure lock 40, a continuously conveying but also pressurized reactor is created. After the material has left the extruder through the material outlet nozzle 74, it is accelerated by a gas blower 78 and the remaining, not yet dissolved non-gaseous particles are separated from the gas. This is done by a separator unit 90 e.g. a cyclone separator. The solids can now be fed back into the twin-screw extruder through the feed-back port 76 where they are further disintegrated. This cycle provides a high yield of levulinic acid, as almost all biomass can be gasified if desired. The pressure can create a natural boundary that prevents the formation of small molecules, and thus de-polymerization and by-products. An adapted reaction pressure can be used to produce targeted levulinic acid. By using a twin screw extruder as a continuously operating pressure reactor, it is possible to realize a process that can compete with batch reactors in terms of yield, but has significant economic advantages. The twin screw extruder is designed to perform two main tasks: A comminution of the material by a suitable screw geometry, and a dissolution of the solid raw material to gaseous wood acids.

The produced gas is condensed to a liquid mixture in e.g. a steam condenser 92 and then passed through a pressure reducer 40. Here, the pressure reducer 40 can equalize the process pressure again. Thus, the output end can also be continuously sealed. This mixture of substances is now separated by distillation, in particular by fractional and vacuum distillation 94, 96 and the levulinic acid is separated. The levulinic acid is then further processed to a bio-polyester by a polymerization unit 98.

Instead of woody biomass, the extrusion may also be carried out in a twin screw extruder with multiple temperature zones, where a starch slurry is preconditioned, extruded, filtered, reboiled, vacuum distilled, condensed, centrifuged, and with the effluent from centrifugation recycled upstream to the preconditioning stage. The raw material is first mixed with water and then the diluted e.g. corn starch is passed through a twin-screw extruder. The mixture is then separated in a filter press and the liquid distilled under vacuum.

### Reference list

- 10: (first) pressure lock
- 20: housing
- 21: cylindrical inner wall of housing
- 22: material feeding terminal of housing
- 23: material feeding opening of housing
- 24: material discharge terminal of housing
- 25: material discharge opening of housing
- 26: first pressure equalizing terminal of housing
- 27: first pressure equalizing opening of first pressure equalizing terminal
- 28: second pressure equalizing terminal of housing
- 29: second pressure equalizing opening of second pressure equalizing terminal
- 30: rotor
- 31: cylindrical outer shape of rotor
- 32: material receiving space of rotor
- 33: primary opening of material receiving space
- 34: secondary/pressure equalizing opening of material receiving space
- 39: rotor drive coupling
- 40: pressure reducer/second pressure lock
- 50: extruding apparatus
- 60: extruder screw of extruding apparatus
- 61: driving rod of extruder screw
- 62: high pressure sealing
- 70: housing of extruding apparatus
- 71: housing interior of extruding apparatus/extruder housing
- 72: lead through opening of extruder housing for driving rod
- 73: material feeding port
- 74: material outlet nozzle
- 75: pressure port
- 76: feedback port/residual non-gaseous particle port
- 77: feeding of (sulfuric) acid
- 78: gas blower
- 80: pressure source
- 90: separator unit/cyclone separator
- 92: steam condenser
- 94: fractional distiller
- 96: vacuum distiller
- 98: polymerization unit
- S1: continuously feeding and processing biomass
- S2: distillation of the gaseous particles
- S3: transformation of produced levulinic acid to monomers
- S4: production of biodegradable polyesters from the monomers

## Claims

1. A pressure lock (10) for material exchange volumes with a pressure difference, the pressure lock comprising:
a housing (20) having a cylindrical inner wall (21),
a rotor (30) having a cylindrical outer shape (31) matching gas tight the cylindrical inner wall (21) of the housing (20) and being rotatable in a rotational direction around a rotational axis within the cylindrical inner wall (21),
wherein the housing (20) has a material feeding terminal (22) with a material feeding opening (23) ending in the cylindrical inner wall, a material discharge terminal (24) with a material discharge opening (25) ending in the cylindrical inner wall offset from the material feeding opening, and a first pressure equalizing terminal (26) with a pressure equalizing opening (27) ending in the cylindrical inner wall offset from the material feeding opening (23) and the material discharge opening (25);
wherein the rotor (30) has formed therein a material receiving space (32) having a primary opening (33) ending at the cylindrical outer shape (31) at a longitudinal position with respect to the rotational axis corresponding to the material feeding opening (23) and the material discharge opening (25), and a secondary opening (34) ending at the cylindrical outer shape (31) at a longitudinal position with respect to the rotational axis corresponding to the first pressure equalizing opening (27),
**characterized in that** upon rotation of the rotor within the housing:
in a first rotational range the primary opening (33) matches the material feeding opening (23) and the secondary opening (34) faces gas tight a closed portion of the cylindrical inner wall (21) of the housing (20),
in a subsequent second rotational range the primary opening (33) and the secondary opening (34) both face gas tight a closed portion of the cylindrical inner wall (21) of the housing (20),
in a subsequent third rotational range the primary opening (33) faces gas tight a closed portion of the cylindrical inner wall (21) of the housing and the secondary opening (34) matches the first pressure equalizing opening (27),
in a subsequent fourth rotational range the primary opening (33) and the secondary opening (34) both face gas tight a closed portion of the cylindrical inner wall (21) of the housing,
in a subsequent fifth rotational range the primary opening (33) matches the material discharge opening (25) and the secondary opening (34) faces gas tight a closed portion of the cylindrical inner wall (21) of the housing (20), and
in a subsequent sixth rotational range the primary opening (33) and the secondary opening (34) both face gas tight a closed portion of the cylindrical inner wall (21) of the housing (20).

2. A pressure lock according to claim 1, wherein the rotor (30) with respect to an axial direction along the rotational axis is fixed with respect to the housing (20) and is rotatable in a rotational direction with respect to the housing (20), wherein the material feeding opening (23) and the material discharge opening (25) with respect to the rotational axis are substantially opposite to each other, in particular at the same axial position along the rotational axis, and offset in rotational direction, in particular offset in rotational direction by 180°.

3. A pressure lock according to any one of claims 1 and 2, wherein the secondary opening (34) with respect to the axial direction is offset from the primary opening (33), in particular the secondary opening (34) with respect to the rotational direction is offset from the primary opening (33), in particular by an angle of approximately 45°.

4. A pressure lock according to any one of claims 1 to 3, wherein the housing (20) further has a second pressure equalizing terminal (28) with a pressure equalizing opening (29) ending in the cylindrical inner wall (21) offset from the material feeding opening (23) and the material discharge opening (25); wherein the secondary opening (34) ends at the cylindrical outer shape (31) at an axial position with respect to the rotational axis corresponding to the first pressure equalizing opening (27) and the second pressure equalizing opening (29), so that upon rotation of the rotor (30) within the housing (20)
in a subsequent seventh rotational range the primary opening (33) faces gas tight a closed portion of the cylindrical inner wall (21) of the housing (20) and the secondary opening (34) matches the second pressure equalizing opening (29),
in a subsequent eighth rotational range the primary opening (33) and the secondary opening (34) both face gas tight a closed portion of the cylindrical inner wall (21) of the housing (20),
wherein after the eighth rotational range the first rotational range follows.

5. An extruding apparatus for high pressure extrusion processing, the apparatus (50) comprising:
at least one extruder screw (60) having a driving rod (61) to be coupled to a drive;
a housing (70) having accommodated in a housing interior (71) the at least one extruder screw (60), the housing having a lead through opening (72) for the driving rod (61), a material feeding port (73) for feeding material to the housing interior (71), a material outlet nozzle (74) for letting out extruded material, and a pressure port (75) for pressurizing the housing interior (71),
a high pressure sealing (62) at the lead through opening (72) for the driving rod (61);
a pressure lock (10) according to any one of the claims 1 to 4 at the material feeding port (73) for feeding material to the housing interior (71) of the extruding apparatus, maintaining the pressure within the housing interior (71) during material feeding.

6. An extruding apparatus according to claim 5, wherein the material discharge terminal (24) is connected gas tight to the material feeding port (73) and which pressure lock (10) is adapted for feeding material into the housing interior (71) of the extruding apparatus (50), wherein the pressure lock (10) is adapted to separate an atmospheric pressure from a process pressure within the housing interior (71) of the extruding apparatus.

7. An extruding apparatus according to any one of claims 5 and 6, further comprising a pressure source (80) being connected to the pressure port (75), wherein the pressure source (80) is a gas pressure source, in particular a nitrogen gas pressure source, in particular a nitrogen gas pressure source being adapted for applying at least 25 bar to the housing interior (71).

8. An extruding apparatus according to any one of claims 5 to 7, wherein the material outlet nozzle (74) for letting out extruded material is adapted for being modified with respect to the outlet geometry of the material outlet nozzle (74), in particular with respect to the fluid flow resistance of the outlet nozzle geometry, in particular with respect to the outlet cross-section of the material outlet nozzle (74).

9. An extruding apparatus according to any one of claims 5 to 8, wherein the extruding apparatus (50) comprises a second pressure lock (40) downstream the material outlet nozzle (74) for letting out material from the housing interior (71) of the extruding apparatus (50), wherein the second pressure lock (40) is adapted for maintaining the pressure within the housing interior (71) of the extruding apparatus (50) during material outlet.

10. An extruding apparatus according to any one of claims 5 to 9, wherein the extruding apparatus (50) further comprises a separator unit (90) downstream the material outlet nozzle (74) for letting out material from the housing interior (71), in particular a cyclone separator being adapted for separating non-gaseous particles from gaseous particles, wherein the housing (70) has a non-gaseous particle port (76) for feeding back the non-gaseous particles from the separator unit (90) to the housing interior (71).

11. An extruding apparatus according to claim 10, wherein the extruding apparatus (50) further comprises a steam condenser (92) downstream the separator unit (90), the steam condenser (92) being adapted for condensing the gaseous particles, and optionally further comprises a distiller (94, 96) downstream the steam condenser (92), in particular a fractional distiller (94) and subsequent a vacuum distiller (96), and optionally further comprises a polymerization unit (98) downstream the distiller (94, 96).

12. Use of the extruding apparatus according to claims 5 to 10 for high pressure extrusion processing of biomass, wherein the biomass is preferably woody biomass.

13. Process for producing a composition from biomass, particularly a chemical compound from biomass, particularly a biopolymer from biomass using the extruding apparatus according to claims 5 to 10, the process comprising
continuously feeding through the pressure lock (10) biomass into the housing interior (71) of the extruding apparatus (50) and processing (S1) of the biomass by at least one of a temperature application to the biomass in the housing interior (71) of the extruding apparatus (50), an acid application to the biomass within the housing interior (71) of the extruding apparatus (50) and a pressure change onto the biomass within the housing interior (71) of the extruding apparatus (50), thus dissolving gas from the biomass;
continuously letting out the dissolved gas from the material outlet nozzle (74), wherein optionally the process further comprises separating non-gaseous particles from gaseous particles and feeding back the non-gaseous particles to the housing interior (71) of the extruding apparatus (50).

14. Process for producing a composition from biomass, particularly a chemical compound from biomass, particularly a biopolymer from biomass according to claim 13, wherein the process further comprises distillation of the gaseous particles (S2), in particular factional distillation of the gaseous particles for obtaining levulinic acid and subsequent vacuum distillation thereof, and optionally subsequent transformation (S3) of the produced levulinic acid to monomers, and further optionally subsequent production (S4) of biodegradable polyesters from the monomers, in particular using at least one of a chemical and an enzymatic polymerization process.

15. Use according to claim 12 or process for producing a composition from biomass, particularly a chemical compound from biomass, particularly a biopolymer from biomass according to any one of claims 13 to 14, wherein the biomass is woody biomass, preferably selected from the group consisting of sawdust, shredded residual wood, wood residues and a composition thereof, wherein the woody biomass in particular has a moisture content of more than 30%, in particular of more than 50%.

## Patentansprüche

1. Druckschleuse (10) für Materialaustauschvolumina mit Druckdifferenz, wobei die Druckschleuse aufweist:
ein Gehäuse (20), welches eine zylindrische Innenwand (21) aufweist,
einen Rotor (30), welcher eine zylindrische Außenform (31) aufweist, die gasdicht an die zylindrische Innenwand (21) des Gehäuses (20) angepasst ist und in einer Drehrichtung um eine Drehachse innerhalb der zylindrischen Innenwand (21) drehbar ist,
wobei das Gehäuse (20) einen Materialzuführterminal (22) mit einer Materialzuführöffnung (23), die in der zylindrischen Innenwand endet, einen Materialaustragsanschluss (24) mit einer Materialaustragsöffnung (25), die in der zylindrischen Innenwand versetzt zur Materialzuführungsöffnung endet, und einen ersten Druckausgleichsanschluss (26) mit einer Druckausgleichsöffnung (27), die in der zylindrischen Innenwand versetzt zur Materialzuführöffnung (23) und zur Materialaustragsöffnung (25) endet, aufweist;
wobei in dem Rotor (30) ein Materialaufnahmeraum (32) mit einer Primäröffnung (33), die an der zylindrischen Außenform (31) an einer Längsposition in Bezug auf die Drehachse endet, die der Materialzuführöffnung (23) und der Materialaustragsöffnung (25) entspricht, und einer Sekundäröffnung (34), die an der zylindrischen Außenform (31) an einer Längsposition in Bezug auf die Drehachse endet, die der ersten Druckausgleichsöffnung (27) entspricht, ausgebildet ist,
**dadurch gekennzeichnet, dass** bei Drehung des Rotors innerhalb des Gehäuses:
in einem ersten Drehbereich die Primäröffnung (33) mit der Materialzuführöffnung (23) übereinstimmt und die Sekundäröffnung (34) gasdicht einem geschlossenen Abschnitt der zylindrischen Innenwand (21) des Gehäuses (20) gegenüberliegt,
in einem anschließenden zweiten Drehbereich die Primäröffnung (33) und die Sekundäröffnung (34) jeweils gasdicht einem geschlossenen Abschnitt der zylindrischen Innenwand (21) des Gehäuses (20) gegenüberliegen,
in einem anschließenden dritten Drehbereich die Primäröffnung (33) gasdicht einem geschlossenen Abschnitt der zylindrischen Innenwand (21) des Gehäuses gegenüberliegt und die Sekundäröffnung (34) mit der ersten Druckausgleichsöffnung (27) übereinstimmt,
in einem anschließenden vierten Drehbereich die Primäröffnung (33) und die Sekundäröffnung (34) jeweils gasdicht einem geschlossenen Abschnitt der zylindrischen Innenwand (21) des Gehäuses gegenüberliegen,
in einem anschließenden fünften Drehbereich die Primäröffnung (33) mit der Materialaustragsöffnung (25) übereinstimmt und die Sekundäröffnung (34) gasdicht einem geschlossenen Abschnitt der zylindrischen Innenwand (21) des Gehäuses (20) gegenüberliegt, und
in einem anschließenden sechsten Drehbereich die Primäröffnung (33) und die Sekundäröffnung (34) jeweils gasdicht einem geschlossenen Abschnitt der zylindrischen Innenwand (21) des Gehäuses (20) gegenüberliegen.

2. Druckschleuse nach Anspruch 1, wobei der Rotor (30) in Bezug auf eine axiale Richtung entlang der Drehachse gegenüber dem Gehäuse (20) feststeht und in einer Drehrichtung gegenüber dem Gehäuse (20) drehbar ist, wobei die Materialzuführöffnung (23) und die Materialaustragsöffnung (25) in Bezug auf die Drehachse im Wesentlichen einander gegenüberliegen, insbesondere an der gleichen axialen Position entlang der Drehachse gegenüberliegen, und in Drehrichtung versetzt sind, insbesondere in Drehrichtung um 180° versetzt sind.

3. Druckschleuse nach einem der Ansprüche 1 und 2, wobei die Sekundäröffnung (34) in Bezug auf die axiale Richtung gegenüber der Primäröffnung (33) versetzt ist, wobei die Sekundäröffnung (34) insbesondere in Bezug auf die Drehrichtung gegenüber der Primäröffnung (33) versetzt ist, insbesondere um einen Winkel von etwa 45° versetzt ist.

4. Druckschleuse nach einem der Ansprüche 1 bis 3, wobei das Gehäuse (20) ferner einen zweiten Druckausgleichsanschluss (28) mit einer Druckausgleichsöffnung (29) aufweist, die in der zylindrischen Innenwand (21) versetzt zur Materialzuführöffnung (23) und zur Materialaustragsöffnung (25) endet; wobei die Sekundäröffnung (34) an der zylindrischen Außenform (31) an einer axialen Position in Bezug auf die Drehachse endet, die der ersten Druckausgleichsöffnung (27) und der zweiten Druckausgleichsöffnung (29) entspricht, so dass bei Drehung des Rotors (30) innerhalb des Gehäuses (20)
in einem anschließenden siebten Drehbereich die Primäröffnung (33) gasdicht einem geschlossenen Abschnitt der zylindrischen Innenwand (21) des Gehäuses (20) gegenüberliegt und die Sekundäröffnung (34) mit der zweiten Druckausgleichsöffnung (29) übereinstimmt,
in einem anschließenden achten Drehbereich die Primäröffnung (33) und die Sekundäröffnung (34) jeweils gasdicht einem geschlossenen Abschnitt der zylindrischen Innenwand (21) des Gehäuses (20) gegenüberliegen,
wobei nach dem achten Drehbereich der erste Drehbereich folgt.

5. Eine Extrudiervorrichtung für die Hochdruck-Extrusionsverarbeitung, wobei die Vorrichtung (50) aufweist:
mindestens eine Extruderschnecke (60) mit einer an einen Antrieb zu koppelnden Antriebsstange (61);
ein Gehäuse (70), in dem die mindestens eine Extruderschnecke (60) in einem Gehäuseinnenraum (71) untergebracht ist, wobei das Gehäuse eine Durchführungsöffnung (72) für die Antriebsstange (61), einen Materialzufuhranschluss (73) zur Materialzuführung in den Gehäuseinnenraum (71), eine Materialauslassdüse (74) zum Auslassen von extrudiertem Material und einen Druckanschluss (75) zur Druckbeaufschlagung des Gehäuseinnenraums (71) aufweist,
eine Hochdruckdichtung (62) an der Durchführungsöffnung (72) für die Antriebsstange (61);
eine Druckschleuse (10) nach einem der Ansprüche 1 bis 4 an dem Materialzufuhranschluss (73) zur Materialzuführung in den Gehäuseinnenraum (71) der Extrudiervorrichtung, die den Druck im Gehäuseinnenraum (71) während der Materialzuführung aufrecht erhält.

6. Extrudiervorrichtung nach Anspruch 5, wobei der Materialaustragsanschluss (24) gasdicht mit dem Materialzufuhranschluss (73) verbunden ist und die Druckschleuse (10) zur Materialzuführung in den Gehäuseinnenraum (71) der Extrudiervorrichtung (50) ausgelegt ist, wobei die Druckschleuse (10) dazu ausgelegt ist, einen atmosphärischen Druck von einem Prozessdruck innerhalb des Gehäuseinnenraums (71) der Extrudiervorrichtung zu trennen.

7. Extrudiervorrichtung nach einem der Ansprüche 5 und 6, ferner umfassend eine Druckquelle (80), die an den Druckanschluss (75) angeschlossen ist, wobei die Druckquelle (80) eine Gasdruckquelle ist, insbesondere eine Stickstoffgasdruckquelle ist, insbesondere eine Stickstoffgasdruckquelle, die für die Beaufschlagung des Gehäuseinnenraums mit mindestens 25 bar ausgelegt ist.

8. Extrudiervorrichtung nach einem der Ansprüche 5 bis 7, wobei die Materialaustrittsdüse (74) zum Auslassen von extrudiertem Material hinsichtlich der Austrittsgeometrie der Materialaustrittsdüse (74) modifizierbar ist, insbesondere hinsichtlich des Fluidströmungswiderstands der Austrittsdüsengeometrie, insbesondere hinsichtlich des Austrittsquerschnitts der Materialaustrittsdüse (74).

9. Extrudiervorrichtung nach einem der Ansprüche 5 bis 8, wobei die Extrudiervorrichtung (50) eine zweite Druckschleuse (40) stromabwärts der Materialaustrittsdüse (74) zum Auslassen von Material aus dem Gehäuseinnenraum (71) der Extrudiervorrichtung (50) aufweist, wobei die zweite Druckschleuse (40) zum Aufrechterhalten des Drucks innerhalb des Gehäuseinnenraums (71) der Extrudiervorrichtung (50) während des Materialaustritts ausgelegt ist.

10. Extrudiervorrichtung nach einem der Ansprüche 5 bis 9, wobei die Extrudiervorrichtung (50) ferner eine Trenneinheit (90) stromabwärts der Materialaustrittsdüse (74) zum Auslassen von Material aus dem Gehäuseinnenraum (71) aufweist, insbesondere ein Zyklonabscheider, der zum Trennen nicht gasförmiger Partikel von gasförmigen Partikeln ausgelegt ist, wobei das Gehäuse (70) einen Anschluss (76) für nicht gasförmige Partikel zum Zurückführen der nicht gasförmigen Partikel von der Trenneinheit (90) in den Gehäuseinnenraum (71) aufweist.

11. Extrudiervorrichtung nach Anspruch 10, wobei die Extrudiervorrichtung (50) ferner einen Dampfkondensator (92) stromabwärts der Trenneinheit (90) aufweist, wobei der Dampfkondensator (92) zum Kondensieren der gasförmigen Partikel ausgelegt ist und optional weiterhin eine Destillieranlage (94, 96) stromabwärts des Dampfkondensators (92) aufweist, insbesondere eine fraktionierte Destillieranlage (94) und anschließend eine Vakuumdestillieranlage (96), und optional weiterhin eine Polymerisationseinheit (98) stromabwärts des Destillators (94, 96) aufweist.

12. Verwendung der Extrudiervorrichtung nach den Ansprüchen 5 bis 10 zur Hochdruckextrusionsverarbeitung von Biomasse, wobei es sich bei der Biomasse vorzugsweise um Holzbiomasse handelt.

13. Verfahren zur Herstellung einer Zusammensetzung aus Biomasse, insbesondere einer chemischen Verbindung aus Biomasse, insbesondere eines Biopolymers aus Biomasse unter Verwendung der Extrudiervorrichtung nach einem der Ansprüche 5 bis 10, wobei das Verfahren aufweist:
kontinuierliches Zuführen von Biomasse durch die Druckschleuse (10) in den Gehäuseinnenraum (71) der Extrudiervorrichtung (50) und Verarbeiten (S1) der Biomasse durch zumindest eine Temperaturbeaufschlagung der Biomasse im Gehäuseinnenraum (71) der Extrudiervorrichtung (50), eine Säurebeaufschlagung auf die Biomasse innerhalb des Gehäuseinnenraums (71) der Extrudiervorrichtung (50) und eine Druckänderung auf die Biomasse innerhalb des Gehäuseinnenraums (71) der Extrudiervorrichtung (50), wodurch Gas aus der Biomasse gelöst wird;
kontinuierliches Auslassen des gelösten Gases aus der Materialaustrittsdüse (74), wobei optional das Verfahren ferner die Trennung nicht gasförmigen Partikeln von gasförmigen Partikeln und das Zurückführen der nicht gasförmigen Partikel in den Gehäuseinnenraum (71) der Extrudiervorrichtung (50) aufweist.

14. Verfahren zur Herstellung einer Zusammensetzung aus Biomasse, insbesondere einer chemischen Verbindung aus Biomasse, insbesondere ein Biopolymer aus Biomasse nach Anspruch 13, wobei das Verfahren ferner eine Destillation der gasförmigen Partikeln (S2) aufweist, insbesondere eine fraktionierte Destillation der gasförmigen Partikel zur Gewinnung von Lävulinsäure und anschließende Vakuumdestillation derselben, und optional eine anschließende Umwandlung (S3) der hergestellten Lävulinsäure in Monomere, und weiterhin optional eine anschließende Herstellung (S4) von biologisch abbaubaren Polyestern aus den Monomeren, insbesondere unter Verwendung mindestens eines chemischen und eines enzymatischen Polymerisationsverfahrens.

15. Verwendung nach Anspruch 12 oder Verfahren zur Herstellung einer Zusammensetzung aus Biomasse, insbesondere einer chemischen Verbindung aus Biomasse, insbesondere ein Biopolymer aus Biomasse nach einem der Ansprüche 13 bis 14, wobei die Biomasse Holzbiomasse ist, vorzugsweise ausgewählt aus der Gruppe bestehend aus Sägespänen, zerkleinertem Restholz, Holzresten und einer Zusammensetzung daraus, wobei die Holzbiomasse insbesondere einen Feuchtigkeitsgehalt von mehr als 30 % aufweist, insbesondere von mehr als 50 %.

## Revendications

1. Sas sous pression (10) pour des volumes d'échange de matériau avec une différence de pression, le sas sous pression comprenant :
un boîtier (20) ayant une paroi intérieure cylindrique (21),
un rotor (30) ayant une forme extérieure cylindrique (31) correspondant de manière étanche aux gaz à la paroi intérieure cylindrique (21) du boîtier (20) et pouvant tourner dans un sens de rotation autour d'un axe de rotation à l'intérieur de la paroi intérieure cylindrique (21),
dans lequel le boîtier (20) comporte un terminal d'alimentation en matériau (22) avec une ouverture d'alimentation en matériau (23) se terminant dans la paroi intérieure cylindrique, un terminal d'évacuation de matériau (24) avec une ouverture d'évacuation de matériau (25) se terminant dans la paroi intérieure cylindrique de manière décalée par rapport à l'ouverture d'alimentation en matériau, et un premier terminal d'égalisation de pression (26) avec une ouverture d'égalisation de pression (27) se terminant dans la paroi intérieure cylindrique de manière décalée par rapport à l'ouverture d'alimentation en matériau (23) et à l'ouverture d'évacuation de matériau (25) ;
dans lequel le rotor (30) présente un espace de réception de matériau (32) ayant une ouverture primaire (33) se terminant sur la forme extérieure cylindrique (31) à une position longitudinale par rapport à l'axe de rotation correspondant à l'ouverture d'alimentation en matériau (23) et à l'ouverture d'évacuation de matériau (25), et une ouverture secondaire (34) se terminant à la forme extérieure cylindrique (31) à une position longitudinale par rapport à l'axe de rotation correspondant à la première ouverture d'égalisation de pression (27),
**caractérisé en ce que**, lors de la rotation du rotor à l'intérieur de boîtier :
dans une première plage de rotation, l'ouverture primaire (33) correspond à l'ouverture d'alimentation en matériau (23) et l'ouverture secondaire (34) fait face de manière étanche aux gaz à une partie fermée de la paroi intérieure cylindrique (21) du boîtier (20),
dans une deuxième plage de rotation suivante, l'ouverture primaire (33) et l'ouverture secondaire (34) font toutes deux face de manière étanche aux gaz à une partie fermée de la paroi intérieure cylindrique (21) du boîtier (20),
dans une troisième plage de rotation suivante, l'ouverture primaire (33) fait face de manière étanche aux gaz à une partie fermée de la paroi intérieure cylindrique (21) du boîtier et l'ouverture secondaire (34) correspond à la première ouverture d'égalisation de pression (27),
dans une quatrième plage de rotation suivante, l'ouverture primaire (33) et l'ouverture secondaire (34) font toutes deux face de manière étanche aux gaz à une partie fermée de la paroi intérieure cylindrique (21) du boîtier,
dans une cinquième plage de rotation suivante, l'ouverture primaire (33) correspond à l'ouverture d'évacuation de matériau (25) et l'ouverture secondaire (34) fait face de manière étanche aux gaz à une partie fermée de la paroi intérieure cylindrique (21) du boîtier (20), et
dans une sixième plage de rotation suivante, l'ouverture primaire (33) et l'ouverture secondaire (34) font toutes deux face de manière étanche aux gaz à une partie fermée de la paroi intérieure cylindrique (21) du boîtier (20).

2. Sas sous pression selon la revendication 1, dans lequel le rotor (30), par rapport à une direction axiale le long de l'axe de rotation, est fixe par rapport au boîtier (20) et est rotatif dans une direction de rotation par rapport au boîtier (20), dans lequel l'ouverture d'alimentation en matériau (23) et l'ouverture d'évacuation de matériau (25), par rapport à l'axe de rotation, sont sensiblement opposées l'une à l'autre, en particulier à la même position axiale le long de l'axe de rotation, et décalées dans la direction de rotation, en particulier décalées dans la direction de rotation de 180°.

3. Sas sous pression selon l'une quelconque des revendications 1 et 2, dans lequel l'ouverture secondaire (34), par rapport à la direction axiale, est décalée par rapport à l'ouverture primaire (33), en particulier l'ouverture secondaire (34), par rapport à la direction de rotation, est décalée par rapport à l'ouverture primaire (33), en particulier d'un angle d'environ 45°.

4. Sas sous pression selon l'une quelconque des revendications 1 à 3, dans lequel le boîtier (20) comporte en outre un deuxième terminal d'égalisation de pression (28) avec une ouverture d'égalisation de pression (29) se terminant dans la paroi intérieure cylindrique (21) de manière décalée par rapport à l'ouverture d'alimentation en matériau (23) et à l'ouverture d'évacuation de matériau (25) ; dans lequel l'ouverture secondaire (34) se termine sur la forme extérieure cylindrique (31) à une position axiale par rapport à l'axe de rotation correspondant à la première ouverture d'égalisation de pression (27) et à la deuxième ouverture d'égalisation de pression (29), de sorte que lors de la rotation du rotor (30) à l'intérieur de boîtier (20) :
dans une septième plage de rotation suivante, l'ouverture primaire (33) fait face de manière étanche aux gaz à une partie fermée de la paroi intérieure cylindrique (21) du boîtier (20) et l'ouverture secondaire (34) correspond à la deuxième ouverture d'égalisation de pression (29),
dans une huitième plage de rotation suivante, l'ouverture primaire (33) et l'ouverture secondaire (34) font toutes deux face de manière étanche aux gaz à une partie fermée de la paroi intérieure cylindrique (21) du boîtier (20),
dans lequel la huitième plage de rotation est suivie de la première plage de rotation.

5. Appareil d'extrusion pour le traitement par extrusion à haute pression, l'appareil (50) comprenant :
au moins une vis d'extrusion (60) comportant une tige d'entraînement (61) destinée à être couplée à un entraînement ;
un boîtier (70) dans un intérieur de boîtier (71) duquel est logée ladite au moins une vis d'extrusion (60), le boîtier comportant une ouverture de passage (72) pour la tige d'entraînement (61), un orifice d'alimentation en matériau (73) pour alimenter en matériau l'intérieur de boîtier (71), une buse de sortie de matériau (74) pour évacuer le matériau extrudé et un orifice de pression (75) pour pressuriser l'intérieur de boîtier (71),
un joint haute pression (62) au niveau de l'ouverture de passage (72) pour la tige d'entraînement (61) ;
un sas sous pression (10) selon l'une quelconque des revendications 1 à 4 au niveau de l'orifice d'alimentation en matériau (73) pour alimenter en matériau l'intérieur de boîtier (71) de l'appareil d'extrusion, en maintenant la pression dans l'intérieur de boîtier (71) pendant l'alimentation en matériau.

6. Appareil d'extrusion selon la revendication 5, dans lequel le terminal d'évacuation de matériau (24) est relié de manière étanche aux gaz à l'orifice d'alimentation en matériau (73) et dont le sas sous pression (10) est adapté à alimenter en matériau l'intérieur de boîtier (71) de l'appareil d'extrusion (50), dans lequel le sas sous pression (10) est adapté à séparer une pression atmosphérique d'une pression de procédé dans l'intérieur de boîtier (71) de l'appareil d'extrusion.

7. Appareil d'extrusion selon l'une quelconque des revendications 5 et 6, comprenant en outre une source de pression (80) reliée à l'orifice de pression (75), dans lequel la source de pression (80) est une source de pression de gaz, en particulier une source de pression d'azote gazeux, en particulier une source de pression d'azote gazeux adaptée à appliquer au moins 25 bars à l'intérieur de boîtier (71).

8. Appareil d'extrusion selon l'une quelconque des revendications 5 à 7, dans lequel la buse de sortie de matériau (74) destinée à évacuer le matériau extrudé est adaptée à être modifiée en ce qui concerne la géométrie de sortie de la buse de sortie de matériau (74), en particulier en ce qui concerne la résistance à l'écoulement de fluide de la géométrie de la buse de sortie, en particulier en ce qui concerne la section transversale de sortie de la buse de sortie de matériau (74).

9. Appareil d'extrusion selon l'une quelconque des revendications 5 à 8, dans lequel l'appareil d'extrusion (50) comprend un deuxième sas sous pression (40) en aval de la buse de sortie de matériau (74) pour évacuer le matériau de l'intérieur de boîtier (71) de l'appareil d'extrusion (50), dans lequel le deuxième sas sous pression (40) est adapté à maintenir la pression dans l'intérieur de boîtier (71) de l'appareil d'extrusion (50) pendant la sortie de matériau.

10. Appareil d'extrusion selon l'une quelconque des revendications 5 à 9, dans lequel l'appareil d'extrusion (50) comprend en outre une unité de séparation (90) en aval de la buse de sortie de matériau (74) pour évacuer le matériau de l'intérieur de boîtier (71), en particulier un séparateur cyclonique adapté à séparer les particules non gazeuses des particules gazeuses, dans lequel le boîtier (70) comporte un port de particules non gazeuses (76) pour renvoyer les particules non gazeuses de l'unité de séparation (90) vers l'intérieur de boîtier (71).

11. Appareil d'extrusion selon la revendication 10, dans lequel l'appareil d'extrusion (50) comprend en outre un condenseur de vapeur (92) en aval de l'unité de séparation (90), le condenseur de vapeur (92) étant adapté à condenser les particules gazeuses, et comprend en outre éventuellement un distillateur (94, 96) en aval du condenseur de vapeur (92), en particulier un distillateur à fractionner (94) et ensuite un distillateur sous vide (96), et comprend en outre éventuellement une unité de polymérisation (98) en aval du distillateur (94, 96).

12. Utilisation de l'appareil d'extrusion selon les revendications 5 à 10 pour le traitement par extrusion à haute pression d'une biomasse, la biomasse étant de préférence une biomasse ligneuse.

13. Procédé de production d'une composition à partir d'une biomasse, en particulier d'un composé chimique à partir d'une biomasse, en particulier d'un biopolymère à partir d'une biomasse à l'aide de l'appareil d'extrusion selon les revendications 5 à 10, le procédé consistant à :
alimenter en continu la biomasse à travers le sas sous pression (10) dans l'intérieur de boîtier (71) de l'appareil d'extrusion (50) et traiter (S1) la biomasse par au moins l'une des méthodes suivantes : application d'une température à la biomasse dans l'intérieur de boîtier (71) de l'appareil d'extrusion (50), application d'un acide à la biomasse dans l'intérieur de boîtier (71) de l'appareil d'extrusion (50) et changement de pression sur la biomasse dans l'intérieur de boîtier (71) de l'appareil d'extrusion (50), dissolvant ainsi le gaz de la biomasse ;
évacuer en continu le gaz dissous à partir de la buse de sortie de matériau (74), le procédé consistant en outre, éventuellement, à séparer les particules non gazeuses des particules gazeuses et à renvoyer les particules non gazeuses vers l'intérieur de boîtier (71) de l'appareil d'extrusion (50).

14. Procédé de production d'une composition à partir d'une biomasse, en particulier d'un composé chimique à partir d'une biomasse, en particulier d'un biopolymère à partir d'une biomasse selon la revendication 13, dans lequel le procédé consiste en outre à distiller les particules gazeuses (S2), en particulier à distiller de manière fractionnée les particules gazeuses pour obtenir de l'acide lévulinique et ensuite à distiller celui-ci sous vide, et éventuellement ensuite à transformer (S3) l'acide lévulinique produit en monomères, et éventuellement ensuite à produire (S4) des polyesters biodégradables à partir des monomères, en particulier en utilisant un procédé de polymérisation chimique et/ou enzymatique.

15. Utilisation selon la revendication 12 ou procédé de production d'une composition à partir d'une biomasse, en particulier d'un composé chimique à partir d'une biomasse, en particulier d'un biopolymère à partir d'une biomasse selon l'une quelconque des revendications 13 à 14, dans laquelle ou lequel la biomasse est une biomasse ligneuse, de préférence choisie dans le groupe constitué par la sciure de bois, le bois résiduel déchiqueté, les résidus de bois et une composition de ceux-ci, dans laquelle la biomasse ligneuse a en particulier une teneur en humidité supérieure à 30 %, en particulier supérieure à 50 %.
